# EUROPÄISCHE PATENTSCHRIFT

(11) **EP 1 588 720 B1**
(45) Veröffentlichungstag und Bekanntmachung des Hinweises auf die Patenterteilung: **08.07.2009**
(21) Anmeldenummer: 04008786.8
(22) Anmeldetag: 13.04.2004
(51) Int. Cl.: A61L 2/10, A61L 2/22, E04H 1/12

(54) **Vorrichtung zur Abtötung pathogener Erreger an Menschen**
Apparatus for killing pathogens on human beings
Appareil pour tuer des pathogènes sur des êtres humains

(43) Veröffentlichungstag der Anmeldung: 26.10.2005
(73) Patentinhaber: Huf, Hans-Joachim, Dr., 55130 Mainz (DE); Ulzhöfer, Hans-Günter, 97877 Wertheim (DE)
(72) Erfinder: Huf, Hans-Joachim, Dr., 55130 Mainz (DE); Ulzhöfer, Hans-Günter, 97877 Wertheim (DE)
(74) Vertreter: Herden, Andreas F.

(56) Entgegenhaltungen:
- EP-A- 0 411 970
- WO-A-00/28552
- WO-A-91/03955
- WO-A-03/077957
- DE-A- 10 113 126
- US-A- 4 348 777
- US-A- 4 366 125
- US-A- 5 533 305

## Beschreibung

Die Erfindung betrifft eine Vorrichtung zur Abtötung pathogener Erreger an Menschen, also im weitesten Sinne ein Desinfektionsverfahren, bei dem pathogene Erreger, wie beispielsweise Bakterien oder Viren abgetötet oder unschädlich gemacht werden.

Desinfektionsverfahren und Desinfektionsmittel sind bekannt. Meist handelt es sich um mikrobiozide Chemikalien. Auch bekannt sind oxidativ wirkende Desinfektionsmittel, wie beispielsweise Wasserstoffperoxid und Peressigsäure. Bekannt ist ferner das Verwenden von elektromagnetischer Strahlung, insbesondere von UV-Strahlung zur Abtötung pathogener Erreger.

Auch eine Kombination aus oxidativ wirkenden Mittel und UV-Strahlung ist bekannt. So beschreibt beispielsweise die DE 199 49 434 eine Kombination von Wasserstoffperoxid und UV-Strahlung zur Desinfizierung von kontaminiertem Wasser. Die WO 00/28552 beschreibt ein Verfahren, bei dem ein photoaktives Aerosol versprüht wird und das zu desinfizierende Objekt mit UV-Stahlung bestrahlt wird.

Die US 4,366,125 beschreibt eine Vorrichtung, bei der ein Folienmaterial mit Wasserstoffperoxid besprüht und mit UV-Strahlung bestrahlt wird. Das Verfahren dient der Desinfektion der Folie.

Nachteilig bei diesen bekannten Desinfektionsverfahren ist, dass zumeist eine Gesundheitsgefahr für Menschen oder Tiere von den Verfahren ausgeht. Von der vorbeugenden Desinfektion beispielsweise großer Personengruppen muss daher in aller Regel abgesehen werden.

Es ist Aufgabe der Erfindung, eine Vorrichtung bereitzustellen, die in besonders einfacher und schneller Weise Menschen desinfiziert. Insbesondere soll eine schnelle Desinfektion einer größeren Anzahl an Personen, zum Beispiel bei biologischen Katastrophenfällen gewährleistet sein.

Die Aufgabe der Erfindung wird in überraschend einfacher Weise eine Vorrichtung gemäß Anspruch 1 erreicht.

Bevorzugte Ausführungsformen und Weiterbildung der Erfindung sind den jeweiligen Unteransprüchen zu entnehmen.

Die Erfindung bezieht sich auf eine Vorrichtung für ein Verfahren zur Desinfektion, Abtötung oder Passivierung pathogener Erreger an Menschen, bei dem der Mensch, mit einem Nebel aus einer Flüssigkeit, die ein oxidativ wirkendes Mittel enthält, besprüht wird und der Organismus oder Gegenstand mit UV-Licht bestrahlt wird.

Das oxidativ wirkende Mittel ist dabei in einer Konzentration vorgesehen, die für Menschen oder Tiere unbedenklich ist.

Die erfindungsgemäße Vorrichtung dient einem Verfahren zur Desinfektion von Menschen und nutzt den Effekt aus, dass oxidative Mittel von UV-Strahlung aktiviert werden können, also dass beispielsweise OH-Radikale gebildet werden, die eine starke oxidative Wirkung haben. Durch die Kombination mit UV-Strahlung ist es überraschenderweise möglich, ein oxidativ wirkendes Mittel in einer Konzentration zu verwenden, die für Menschen unschädlich ist und dennoch einen hohen Desinfektionsgrad zu erreichen. Bei Durchführung des Verfahrens wird zunächst der Mensch mit einem Nebel besprüht, es wird dabei eine Flüssigkeit fein verteilt in dem Raumabschnitt versprüht, in dem sich der Mensch befindet. Dadurch wird der Mensch gleichmäßig mit Tröpfchen besprüht und beaufschlagt. Allein das oxidativ wirkende Mittel hat schon eine gewisse desinfizierende Wirkung, die allerdings oft noch nicht ausreicht, um einen hinreichenden Abtötungsgrad pathogener Erreger zu erreichen. Nach dem Besprühen wird der Mensch mit UV-Licht bestrahlt. Auch das UV-Licht hat für sich genommen schon eine desinfizierende Wirkung. Letztlich wird aber nur durch die Kombination von oxidativ wirkendem Mittel und der anschließenden Aktivierung durch UV-Licht eine hinreichende Desinfektionswirkung, ein Synergieeffekt, erreicht.
Eine Bestrahlung mit UV-Licht ist auch schon während der Sprühphase denkbar.

Bei einer besonderen Ausführungsform des Verfahrens wird eine Peressigsäure-Lösung mit einer Konzentration von unter 0,8 % (jeweils Volumenprozent), bevorzugt von unter 0,6 %, besonders bevorzugt von unter 0,4 % als oxidativ wirkendes Mittel verwendet. Peressigsäure-Konzentrationen von unter 0,8% sind gesundheitlich unbedenklich, geht man mit der Konzentration sogar auf unter 0,4%, sind Nebenwirkungen wie beispielsweise Reizungen der Haut und Atemwege praktisch ausgeschlossen, so dass diese Peressigsäure-Lösung unbedenklich auch zur Behandlung von Menschen verwendet werden kann. Erstaunlicherweise reicht aber bereits in Kombination mit dem UV-Licht eine Konzentration von unter 0,8 %, um eine hinreichende desinfizierende Wirkung zu erreichen.

Alternativ zur Peressigsäure bietet sich bei einer besonderen Ausführungsform eines Verfahrens auch eine Lösung mit Wasserstoffperoxid an, das auch in einer geeigneten geringen Konzentration verwendet werden kann.

Bei einer besonderen Ausführungsform der Erfindung bewegt sich der Mensch zuerst an einer Einrichtung zum Versprühen der Flüssigkeit und danach an einer UV-Lichtquelle zur Bestrahlung entlang. So ist es möglich, Menschen nacheinander zu desinfizieren. Insbesondere können so größere Anzahlen an Menschen schnell nacheinander desinfiziert werden.

Denkbar ist natürlich auch, dass sich der Mensch aus eigener Kraft an der Einrichtung zum Versprühen und an der UV-Lichtquelle entlang bewegt.

Bei einer weiteren besonderen Ausführungsform der Erfindung weist der Nebel eine durchschnittliche Tröpfchengröße zwischen 1 *µ*m und 50 *µ*m, bevorzugt zwischen 2 *µ*m und 15 *µ*m und besonders bevorzugt zwischen 4 *µ*m und 12 *µ*m auf.

Insbesondere bei der Desinfektion von Personen hat sich herausgestellt, dass eine Tröpfchengröße zwischen 4 *µ*m und 12 *µ*m besonders geeignet ist, um einen gleichmäßigen Film auf der Oberfläche zu bilden. Größere Tröpfchengrößen sind insbesondere geeignet, um sich schnell bewegende Gegenstände und Personen zu behandeln. Falls also ein sehr hoher Anlagendurchsatz gefordert ist, kann daher auch eine größere Tröpfchengröße von Vorteil sein.

Als Bestrahlungsdosis hat sich besonders geeignet eine Dosis zwischen 0,3 und 1,0 mWs/cm² herausgestellt, insbesondere eine Dosis zwischen 0,4 und 0,6 mWs/cm² ist zur Behandlung von Menschen besonders geeignet. Es handelt sich hierbei um eine Dosis, die bereits zur hinreichenden Desinfektion geeignet ist, auf der anderen Seite aber auch keine Strahlungsschäden wie etwa Sonnenbrand verursacht. Beispielsweise kann bei einer Bestrahlungsdosis von 0,5 mWs/cm² eine Person bis zu zwölfmal mit einem Verfahren desinfiziert werden, bevor ein Grenzwert von 6 mWs/cm² für die Bestrahlung von Haut mit UV-Strahlung erreicht wird. Ein solches Verfahren ist somit auch in Bezug auf die UV-Strahlungsdosis unbedenklich.

Bei einer weiteren bevorzugten Ausführungsform der Erfindung beträgt die Bestrahlungsdauer zwischen einer und zehn Sekunden, besonders bevorzugt zwischen vier und acht Sekunden. So ist ein hoher Anlagendurchsatz gewährleistet. Es ist dabei gleichzeitig ein Strahlungsfluss von etwa 0,1 mWs/cm² ausreichend.

Für die Bestrahlung als besonders vorteilhaft herausgestellt hat sich eine Wellenlänge von 240 bis 280 Nanometern, insbesondere eine Wellenlänge von 250 bis 270 Nanometern. So hat man herausgefunden, dass für die Bakterientötung bei 254 Nanometern ein Maximum erreicht wird. Der bevorzugte Wellenlängenbereich liegt um diesen Wert herum.

Geeigneterweise wird ein Mensch desinfiziert, der während der Durchführung eines Verfahrens unter Verwendung der erfindungsgemäßen Vorrichtung eine UV-Strahlung absorbierende Brille trägt. So ist gewährleistet, dass es nicht zu UV-Strahlungsschäden an den Augen oder zur Blendung kommen kann.

Die Erfindung betrifft eine Vorrichtung zur Desinfektion, Abtötung oder Passivierung pathogener Erreger an Menschen mit einem ersten Bereich, in dem zumindest eine Vorrichtung zum Versprühen einer Flüssigkeit vorgesehen ist, die ein oxidativ wirkendes Mittel enthält und einem zweiten Bereich mit zumindest einer UV-Lichtquelle. Das Verfahren kann in einer erfindungsgemäßen Vorrichtung integriert durchgeführt werden. Vorzugsweise ist das oxidativ wirkende Mittel in einer für Menschen unbedenklichen wirkenden Konzentration vorgesehen.

Bei einer Weiterbildung der Erfindung ist der erste Bereich von dem zweiten Bereich räumlich nicht getrennt. So kann die gesamte Desinfektion in einer Anlage durchgeführt werden. Der Mensch wird also beispielsweise in einen ersten Bereich verbracht, wo er mit einem oxidativ wirkenden Mittel besprüht wird. Sodann wird er in einen zweiten Bereich verbracht, in dem er mit UV-Strahlung bestahlt wird. Diese Ausgestaltungsform hat den Vorteil, dass beispielsweise in dem ersten Bereich eine Wanne zum Auffangen von Flüssigkeit vorgesehen sein kann, anschließend kann sich der Mensch in einen zweiten Bereich bewegen, in dem UV-Lichtquellen angeordnet sind. Konstruktiv kann jeder Bereich unterschiedlichen Anforderungen angepasst werden. So muss beispielsweise der erste Bereich resistent gegen oxidativ wirkende Mittel sein, während der zweite Bereich aus UVstrahlungsresistenten Materialien aufgebaut sein muss.

Bevorzugterweise ist die erfindungsgemäße Vorrichtung zur Desinfektion größerer Personenmengen geeignet, beispielsweise auf Flughäfen, wenn beispielsweise ein Flugzeug aus einem Seuchengebiet eintrifft oder zur Desinfektion von Tierherden, beispielsweise einer Tierherde, die in ein Schiff verladen werden soll.

So kann mit der erfindungsgemäßen Vorrichtung die Ausbreitung von ansteckenden Krankheiten verhindert werden.

Bei einer Weiterbildung der Erfindung ist in die Vorrichtung ein Tank zur Aufnahme der Flüssigkeit des oxidativ wirkenden Mittels integriert. So muss kein Anschluss für die Flüssigkeit vorgesehen sein. Es hat sich gezeigt, dass bereits geringe zu versprühende Mengen ausreichen, um eine angemessene Desinfektion zu erreichen, so dass keine Zuführung von außen erforderlich ist. Insbesondere bei einer mobilen Bauweise der erfindungsgemäßen Vorrichtung ist ein Tank von Vorteil.

Bei einer weiteren vorteilhaften Ausführungsform der Vorrichtung ist eine Brille vorgesehen, die ein zu desinfizierender Mensch tragen kann. Diese Brille verhindert zu einen, dass das oxidativ wirkende Mittel in die Augen des Benutzers gesprüht wird, zum anderen schützt die Brille vor UV-Strahlung im zweiten Bereich der Vorrichtung.

Eine Weiterbildung der Erfindung sieht eine Vorrichtung vor, die zum mobilen Transport ausgebildet ist. Es ist denkbar, eine Vorrichtung beispielsweise in einen Container zu integrieren, der leicht transportiert werden kann. Im Katastrophenfall kann so die erfindungsgemäße Vorrichtung an den"Katastrophenort verbracht werden. Auch ist denkbar, die Vorrichtung in einem Fahrzeug oder Flugzeug zu integrieren.

Bei einer weiteren Ausführungsform der Erfindung ist die Vorrichtung demontierbar. Es ist so denkbar, eine zerlegbar Vorrichtung vorzusehen, die wenig Stauraum einnimmt und so zum Beispiel leicht in einem Flugzeug transportiert werden kann und somit noch leichter in ein Katastrophengebiet verbringbar ist.

Bei einer Weiterbildung der Erfindung ist die Vorrichtung teleskopartig zusammenschiebbar, insbesondere ist vorgesehen, dass der zweite Bereich zumindest teilweise in den ersten Bereich einschiebbar ist. Es hat sich herausgestellt, dass der zweite Bereich mit den UV-Lampen ein größeres Volumen einnimmt als der erste Bereich. Es bietet sich beispielsweise daher an, den zweiten Bereich teleskopartig auszugestalten und in den ersten Bereich einzuschieben.

Besonders vorteilhaft für den ersten Bereich, in dem das oxidativ wirkende Mittel versprüht wird, ist eine Ausgestaltung aus Edelstahl. Dieses Material zeichnet sich durch eine hohe Beständigkeit gegenüber oxidativ wirkenden Mitteln aus.

Auch der zweite Bereich kann aus Edelstahl gefertigt sein. Hier ist aber beispielsweise auch Aluminium ein sehr vorteilhafter Werkstoff, der besonders leicht und UV-strahlungsresistent ist. Kunststoffe haben dagegen zumeist den Nachteil, dass sie nicht UV-beständig sind. Denkbar wäre allenfalls, einen Kunststoff-Verbundmaterial mit einer UV-undurchlässigen Schutzschicht zu versehen.

Bei einer weiteren bevorzugten Ausführungsform der Erfindung ist die Vorrichtung durch Türen an jeweils dem ersten und zweiten Bereich verschließbar. So kann verhindert werden, dass das oxidativ wirkende Mittel oder die UV-Strahlung nach außen tritt. Bei den Türen kann es sich beispielsweise um Schiebetüren handeln, aber auch ein Vorhang ist insbesondere bei dem zweiten Bereich in vorteilhafter Weise denkbar.

Bei einer Weiterbildung der Erfindung werden die Türen über einen Sensor automatisch gesteuert. So braucht beispielsweise eine zu desinfizierende Person keinen Griff oder ähnliches anzufassen, wovon wiederum eine Infektionsgefahr ausgehen könnte. Statt dessen geht die Tür automatisch bei Betreten der Vorrichtung auf und verschließt sich automatisch wieder hinter der Person.

Eine weitere vorteilhafte Ausführungsform der Erfindung weist einen Sensor zur Steuerung des Sprühvorgangs oder einen Sensor zur Steuerung der UV-Strahlung aus. Es kann sich dabei beispielsweise um eine Lichtschranke oder einen Bewegungssensor handeln, der den Sprühvorgang oder die UV-Strahlung aktiviert, sobald die Person den jeweiligen Bereich betritt. So ist eine vollautomatische Anlage denkbar, ohne dass Bedienpersonal oder die zu desinfizierende Person selbst die Vorrichtung bedienen muss.

Vorteilhaft ist dieser Sensor vor direkter UV-Strahlung geschützt angeordnet. Da viele Sensoren Kunststoffe enthalten, könnte die UV-Strahlung die Lebensdauer des Sensors beeinträchtigen.

Bei einer vorteilhaften Ausführungsform der Erfindung erkennen ein oder mehrere Sensor das Erreichen des ersten Bereiches, des zweiten Bereiches oder das Verlassen der Vorrichtung. Sprühvorgang oder UV-Strahlung können so beispielsweise gesteuert werden, dass sie nur aktiv sind, wenn sich eine Person im jeweiligen Bereich befindet. Besonders ein automatisches An und Ausschalten des Sprühvorgangs ist vorteilhaft, da so die Flüssigkeit mit dem oxidativ wirkenden Mittel gespart wird.

Eine Weiterbildung der Erfindung weist eine akustische oder optische Führungseinrichtung auf. Denkbar ist hierbei beispielsweise eine sprachgesteuerte Führung oder eine Führung mittels einer Signaleinrichtung, die anzeigt, wann beispielsweise die nächste Person die Vorrichtung betreten kann, wann der jeweilige Bereich verlassen werden soll oder wann die Vorrichtung verlassen werden soll.

Bei einer weiteren bevorzugten Ausgestaltungsform der Erfindung weist die Vorrichtung eine Einrichtung zum automatischen Abschalten des Sprühvorgangs oder der UV-Bestrahlung nach einer vorgegebenen Zeit auf. Eine solche Vorrichtung, beispielsweise eine Zeitschaltuhr ist insbesondere für den Sprühvorgang wichtig, da sonst Desinfektionsmittel unnötig versprüht wird oder es beispielsweise zu einer Durchnässung der Kleidung des Bedieners kommen kann, oder falls Personen, die Anlage, aus welchen Gründen auch immer, verlassen.

Eine Weiterbildung der Erfindung weist einen Transponder für eine Karte, insbesondere für eine Strichcode-, Magnet-oder Chipkarte auf. Diese Ausführungsform ist insbesondere für sicherheitssensible Bereiche besonders geeignet, beispielsweise den OP-Bereich eines Krankenhauses oder ein Labor, in dem Personen, die das Labor betreten, möglicherweise kontaminiert werden. Hier ist es denkbar, der jeweiligen Person eine Karte mitzugeben, mit der sie die Vorrichtung passiert. Anschließend kann entweder die Karte ausgelesen werden oder einer auf der Karte integrierten Speichereinrichtung übermittelt werden, dass die Vorrichtung passiert wurde. Mittels dieser ausgelesenen oder übermittelten Daten kann beispielsweise sichergestellt werden, dass eine Person erst nach Passieren der erfindungsgemäßen Vorrichtung einen kontaminierten Bereich verlassen kann beziehungsweise erst nach Passieren der Vorrichtung einen sicherheitsrelevanten Bereich betreten kann. Vorteilhafterweise kann die Karte auch gleichzeitig Bestandteil eines anderen Sicherheitssystems wie einer Schließanlage sein. Durch diese Registrierung kann der Nachweis nachträglich erbracht werden, dass die Person oder Sache mit dem erfindungsgemäßen Verfahren beziehungsweise der erfindungsgemäßen Vorrichtung behandelt wurde. Dies ist insbesondere wichtig für Versicherungsfragen oder das Nachvollziehen möglicher Infektionswege.

Bei einer Weiterbildung der Erfindung weist die Vorrichtung ein Förderband zum Transportieren der Organismen oder Gegenstände auf. So ist ein automatisches Durchlaufen der erfindungsgemäßen Vorrichtung gesichert. Insbesondere bei Tieren ist statt einem Transportband aber auch eine ähnliche Führungseinrichtung, wie beispielsweise ein Gitter, das durch die Vorrichtung läuft denkbar, bei dem das Tier dazu gezwungen wird mitzulaufen. Durch den automatischen Transport ist gewährleistet, dass die Vorrichtung auch etwa innerhalb der vorgegeben Zeit durchlaufen wird.

Eine bevorzugte Ausführungsform der Erfindung weist nacheinander angeordnete Bereiche mit Sprüheinrichtung und UV-Lichtquelle auf, wobei zwischen dem ersten und zweiten Bereich keine Sichtschutzwand vorgesehen ist. Es hat sich als besonders vorteilhaft erwiesen, dass durch das UV-Licht zumindest im zweiten Bereich nahezu alle in der Luft befindlichen Keime abgetötet werden. Sind nun erster und zweiter Bereich nacheinander angeordnet und befindet sich keine Sichtschutzwand dazwischen, wird schon durch die Zirkulation der Luft zwischen erstem und zweitem Bereich erreicht, dass in der gesamten Anlage sich keine Keime in der Luft befinden.

Die Vorrichtung gemäß der Erfindung weist auf der einen Seite einen Eingang und auf der gegenüberliegenden Seite einen Ausgang auf. So kann die Vorrichtung in einer Richtung passiert werden. Dies ist insbesondere bei der Desinfektion einer größeren Personen anzahl vorteilhaft.

Bei einer Weiterbildung obiger Ausführungsform der Erfindung ist gegenüber dem Eingang oder gegenüber dem Ausgang eine Sichtschutzwand beziehungsweise ein Sichtschutzvorhang angeordnet. Dieser Sichtschutzvorhang verhindert, dass Personen von vorne oder hinten in die Vorrichtung hineinsehen können. Insbesondere die ungewollte Bestrahlung von Personen mit UV-Licht wird so verhindert.

Vorteilhafterweise ist die Sichtschutzwand oder der Sichtschutzvorhang zurückfahrbar oder zurückschiebbar ausgebildet. Es hat sich insbesondere gezeigt, dass wenn die Sichtschutzwand hinreichend nahe gegenüber dem Ein- oder dem Ausgang positioniert ist, Rollstuhlfahrer nicht durch die Vorrichtung fahren können. Daher ist es von Vorteil, wenn die Sichtschutzwand oder der Sichtschutzvorhang zurückschiebbar ausbildet ist. Vorteilhaft ist hier insbesondere ein automatisches Wiederzurückfahren der Sichtschutzwand in die Ausgangsposition nach einem Durchgang der Anlage. So wird verhindert, dass aus Bequemlichkeit die Sichtschutzwand oder der Sichtschutzvorhang dauerhaft zurückgeschoben bleibt.

Gemäß der Erfindung ist die Vorrichtung zur Desinfektion von Menschen ausgebildet und weist zumindest im ersten Bereich ein beidseitiges Geländer auf. Dieses Geländer ist so hoch angeordnet, dass der zu desinfizierende Mensch die Arme abwinkeln muss. So wird erreicht, dass auch auf der Unterseite der Arme eine ausreichende Menge des oxidativ wirkenden Mittels anhaftet. Vorteilhafterweise geht das beidseitige Geländer auch in dem zweiten Bereich weiter, so dass hier auch eine Bestrahlung unter den Armen erfolgt.

Bei einer Weiterbildung der Erfindung weist die Vorrichtung ein Geländer auf, durch das über Lüfter Raumluft leitbar ist. Insbesondere im zweiten Bereich neigt ein Geländer dazu durch die absorbierte UV-Strahlung warm zu werden, während im ersten Bereich die Flüssigkeit zu einer Benetzung des Geländers mit Nässe führt. Durch einen Lüfter, der Raumluft aus der Vorrichtung durch das Geländer bläst, wird zum einen erreicht, dass sich das Geländer im zweiten Bereich abkühlt, wohingegen der erste Bereich erwärmt wird und so im Wesentlichen trocken bleibt. Denkbar ist vor allem auch, im Geländer Austrittsöffnungen für die Luft vorzusehen. Die Luft, die durch die UV-Strahlung im zweiten Bereich im Wesentlichen keimfrei ist, führt somit zusätzlich zu einer Trocknung zur Desinfizierung des Geländers. Bevorzugterweise kann der Lüfter in dem Geländer integriert sein.

Vorteilhafterweise hat der zweite Bereich der Vorrichtung eine wesentlich größere Länge als der erste Bereich der Vorrichtung. So ist es möglich, dass beim gleichmäßigen Durchschreiten der Vorrichtung die Sprühzeit, die meistens wenigstens kürzer ist als die Bestrahlungszeit, und die Bestrahlungszeit aufeinander abgestimmt sind.

Bei einer Weiterbildung der Erfindung weist die Vorrichtung eine unabhängige Stromversorgung zum Betrieb der UV-Lichtquelle auf. So ist es möglich, die Vorrichtung autonom zu betreiben. Beispielsweise denkbar ist ein Stromgenerator, der die Lampen betreibt. Im Katastrophenfall ist eine solche Desinfektionseinrichtung so nicht auf eine Stromversorgung angewiesen. Denkbar ist auch mit der Stromversorgung eine Pumpe zum Versprühen des Desinfektionsmittels oder den zuvor erwähnten Lüfter zu betreiben.

Bei einer Weiterbildung der Erfindung ist die Vorrichtung als Schleuse in einem Operationssaal ausgebildet. Die Vorrichtung ist so in ein Schleusenelement integriert. Somit wird erreicht, dass der Operationssaal, der im Wesentlichen keimfrei sein sollte, nur von desinfizierten Personen betreten wird.

Vorteilhafterweise ist auch denkbar, die Vorrichtung in einer Brücke, wie sie bei größeren Flughäfen typischerweise vorgesehen sind, um am Flugzeug anzudocken, durch die die Passagiere das Flugzeug verlassen, zu integrieren. So können beispielsweise alle Insassen, die ein Flugzeug verlassen, mit der erfindungsgemäßen Vorrichtung desinfiziert werden.

Alternativ bietet sich an, die Vorrichtung beispielsweise als aufblasbares Tor an einem Fortbewegungsmittel insbesondere einem Flugzeug oder einem Fahrzeug zum Transport von kranken Menschen oder Tieren zu integrieren. So kann das Infektionspotential insbesondere von kranken Personen und deren Behandlungspersonal verringert werden.

Die Erfindung betrifft ferner die Verwendung einer erfindungsgemäßen Vorrichtung oder eines erfindungsgemäßen Verfahrens zur Desinfektion von Menschen insbesondere im Krankenhaus, im OP, in Sportstadien, in Sportanlagen, in Kuranlagen, auf Flugplätzen, insbesondere auf der Gangway eines Flughafens, in Saunen, in Schlachthöfen, in Bunkern oder in Massagesalons, Massagepraxen und ähnlichen Dienstleistungsbetrieben. Gerade in dieses Bereichen ist oft eine hohe Infektionsgefahr vorhanden, eine Desinfektion erfolgt aber falls überhaupt, mittels mikrobiozider oder fungizider Chemikalien, von denen meist Nebenwirkungen ausgehen. Überraschenderweise ist mit dem erfindungsgemäßen Verfahren oder der erfindungsgemäßen Vorrichtung eine Desinfektion von Menschen oder Tieren eine gesundheitlich völlig unbedenkliche Desinfektion möglich. Vor allem durch eine niedrige Konzentration eines oxidativen Mittels wird dieser Vorteil aufgrund des Synergieeffektes erreicht. Durch eine Aktivierung mit UV-Strahlung ist eine gesundheitlich völlig unbedenkliche Konzentration ausreichend.

Die Vorrichtung eignet sich neben der Deinsinfektion von Menschen und Gegenständen vor allem auch zur Desinfektion von Tieren, beispielsweise Schafherden oder Geflügel, von dem oft eine besonders hohe Infektionsgefahr ausgeht. Aufgrund der gesundheitlichen Unbedenklichkeit, die sogar für dem Umgang mit dem oxidativ wirkenden Mittel durch das Bedienpersonal gilt, gibt es einen breiten Verwendungsbereich. Selbst die Desinfektion von Lebensmitteln wie beispielsweise Obst ist vorgesehen.

Die erfindungsgemäße Vorrichtung sollte insbesondere immer da verwendet werden, wo eine große Keimfreiheit oder ein besonders hohes Infektionspotential vorhanden ist, also beispielsweise in Katastrophengebieten, an Flughäfen, Häfen oder Landesgrenzen.

Insbesondere ist die Verwendung der Vorrichtung zur Desinfektion von Gepäckstücken, insbesondere auf Flughäfen, oder von Geld vorgesehen. Gerade von diesen Gegenständen geht erfahrungsgemäß eine besonders hohe Infektionsgefahr aus.

Zur Verwendung in einer erfindungsgemäßen Vorrichtung ist eine Brille vorgesehen. Die Brille weist eine UVabsorbierende Folie als Sichtfeld auf. Eine solche Folie kann beispielsweise als Einwegbrille verwendet werden.

Bevorzugterweise ist die Folie in etwa rechteckig geformt und weist randseitig Schlitze mit Aussparungen zur Anbringung eines elastischen Bandes aus. So ist es beispielsweise möglich, eine Folie zu stanzen und diese dann mit einem handelsüblichen Gummiband zu versehen und als Brille zu verwenden. Diese Konstruktion hat insbesondere den Vorteil, dass sie zumindest bei Menschen an der Stirn einen im Wesentlichen wasserdichten Abschluss bildet, so dass bei der Durchführung des erfindungsgemäßen Verfahrens verhindert wird, dass die oxidativ wirkende Flüssigkeit oder mit der Flüssigkeit vermischte Schweißperlen in die Augen des Benutzers tropfen können. Unter rechteckig im Sinne der Erfindung wird auch eine im wesentlichen rechteckige Grundform mit abgerundeten Kanten oder kurvenförmigen Seiten verstanden.

Bei einer vorteilhaften Weiterbildung der Erfindung ist die Brille Teil eines mehrere Brillen umfassenden Bandes und durch eine Sollbruchlinie, insbesondere eine Perforationslinie, von anderen Brillen des Bandes trennbar.

Somit wäre es beispielsweise möglich, Brillen aus einer kontinuierlichen Materialbahn zu fertigen. Diese könnten dann beispielsweise als Rolle aufgewickelt werden und in einen Spender eingelegt werden, der jeweils eine Brille freigibt. Die Brillen könnten dann abgerissen und vom Benutzer der erfindungsgemäßen Desinfektionsvorrichtung mit einem Gummiband versehen werden und getragen werden. Auch ein Spender, der einzelne übereinander gestapelte Brillenfolien enthält, ist denkbar.

Bevorzugterweise hat die UV-absorbierende Folie eine Dicke zwischen 50 *µ*m bis 200 *µ*m, besonders bevorzugt zwischen 120 *µ*m und 180 *µ*m. Eine solche Folie hat eine hinreichende Flexibilität. Sie kann beispielsweise bei Brillenträgern über einem Brillengestell darüber getragen werden.

Die Erfindung soll im Folgenden anhand von Ausführungsbeispielen dargestellt in den Figuren 1 bis 9 näher erläutert werden.
- Fig. 1: zeigt eine schematische Seitenansicht einer erfindungsgemäßen Vorrichtung, hier ausgestaltet insbesondere zur Desinfektion von Menschen,
- Fig. 2: zeigt eine schematische Draufsicht dieser Vorrichtung von oben,
- Fig. 3: zeigt eine schematische Draufsicht dieser Vorrichtung von vorne,
- Fig. 7: zeigt eine schematische Darstellung einer erfindungsgemäßen Brille,
- Fig. 8: zeigt eine weitere schematische Darstellung einer erfindungsgemäßen Desinfektionskabine zur Desinfektion von Personen,
- Fig. 9: zeigt eine schematische Darstellung von in ein Flugzeug oder in eine Brücke eines Flughafens integrierte Vorrichtung zur Desinfektion von Menschen.
- Fig. 10: zeigt eine schematische Darstellung des ersten Bereiches der erfindungsgemäßen Vorrichtung.
- Fig. 11: zeigt eine schematische Darstellung des zweiten Bereiches der erfindungsgemäßen Vorrichtung

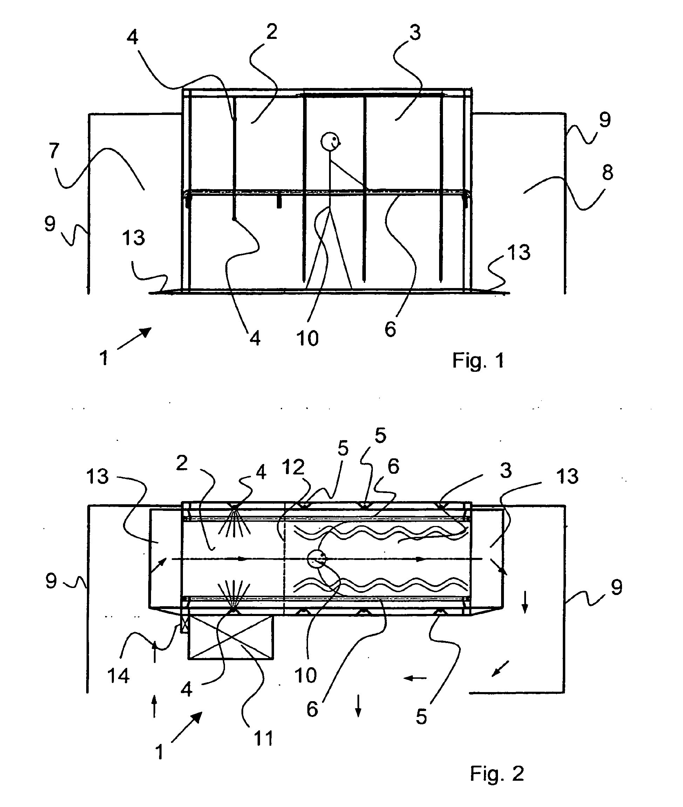

Fig. 1 zeigt eine schematische Darstellung einer erfindungsgemäßen Vorrichtung 1 zur Desinfektion insbesondere einer Person 10. Die Vorrichtung weist einen ersten Bereich 2 und einen zweiten Bereich 3 auf. Der erste Bereich dient der Benetzung der Person 10 mittels eines Sprühnebels aus einer Peressigsäurelösung. Dazu sind im ersten Bereich 2 Sprühköpfe 4 vorgesehen. Der zweite Bereich 3 weist UV-Lampen (nicht dargestellt) auf, mit denen die Person bestrahlt wird. Ferner ist zu erkennen ein Eingangsbereich 7 und ein Ausgangsbereich 8. Vor dem Eingangsbereich 7 und dem Ausgangsbereich 8 befindet sich eine Sichtschutzwand 9. Diese Sichtschutzwand 9 verhindert, dass Personen von vorne oder hinten während des Betriebs beziehungsweise bei eingeschalteter UV-Lampe in die Vorrichtung 1 schauen können. Im Betrieb läuft die zu desinfizierende Person 10 zunächst durch den Eingangsbereich 7. Die Person 10 betritt den ersten Bereich 2 der Desinfektionsvorrichtung 1. Auf beiden Seiten ist ein Geländer 6 angeordnet, das so hoch ist, dass die Person 10 die Arme anwinkeln muss, so dass aus den Sprühköpfen 4 das Desinfektionsmittel am gesamten Körper verteilt wird.

Im ersten Bereich 2 weist die erfindungsgemäße Vorrichtung eine Edelstahlwanne (nicht dargestellt) auf, welche daneben gesprühtes Desinfektionsmittel auffängt. Nach Durchschreiten des ersten Bereiches 2 betritt die Person 10 den zweiten Bereich 3, in dem UV-Lampen angeordnet sind.

Hier wird die Peressigsäure aktiviert, das heißt sie spaltet OH-Radikale ab, was zur Desinfektion gemäß des erfindungsgemäßen Verfahrens führt. Der zweite Bereich 3 ist länger als der erste Bereich 2, da in diesem Bereich eine höhere Verweildauer sinnvoll ist. Nach Durchschreiten des zweiten Bereiches 3 tritt die Person 10 über den Ausgangsbereich 8 und kann so die erfindungsgemäße Vorrichtung verlassen. Ferner weist die Vorrichtung an Eingangs- und Ausgangsseite noch eine Rampe 13 auf, um auch Rollstuhlfahrern das Befahren der erfindungsgemäßen Vorrichtung zu ermöglichen. Diese schematisch dargestellte Vorrichtung ist als mobile Einheit ausgestaltet, kann also relativ leicht transportiert werden und so beispielsweise in Katastrophengebieten oder an Flughäfen, Häfen oder Landesgrenzen aufgestellt werden.

Fig. 2 zeig eine schematische Draufsicht der in Fig. 1 gezeigten Vorrichtung 1. Zu sehen ist auch hier der erste Bereich 2, in dem Sprühköpfe 4 zur Verteilung des Desinfektionsmittels vorgesehen sind. Im zweiten Bereich 3 sind UV-Lampen 5 vorgesehen. Die Person 10 läuft über die Rampe 13, betritt den ersten Bereich 2, dann den zweiten Bereich 3 und verlässt über eine weitere Rampe 13 den zweiten Bereich 3 und somit die Vorrichtung. Die Laufrichtung ist mit Pfeilen eingezeichnet. Zu erkennen ist das Geländer 6, der Schaltschrank 11, der in diesem Fall auch die nötige Elektrik für eine automatische Führungseinrichtung einschließlich der Pumpe (nicht dargestellt) zum Versprühen der oxidativ wirkenden Flüssigkeit umfasst. Die Vorrichtung ist mit einer optischen Führungseinrichtung in Form einer Signallampe 14 ausgestaltet. Auch hier sind die Trennwände 9 zu erkennen, die verhindern, dass Personen im Betrieb von vorne oder hinten in die Vorrichtung 1 schauen können. Auf der rechten Seite, wo die Sichtschutzwand 9 gegenüber dem zweiten Bereich 3 mit den UV-Lampen angeordnet ist, ist die Sichtschutzwand 9 zusätzlich um die Ecke gezogen. So ist ausgeschlossen, dass eine Person von außen schräg in das UV-Licht blicken kann.

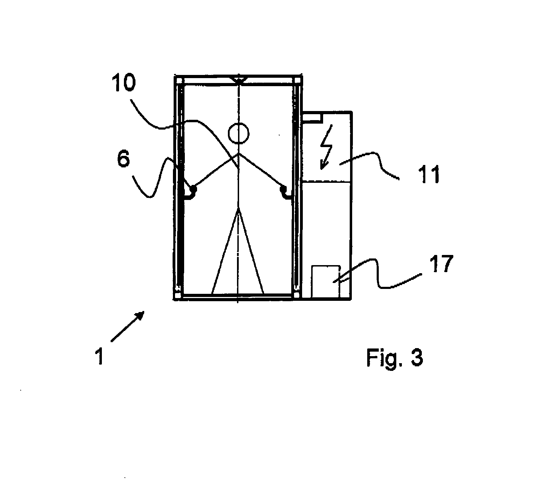

Fig. 3 zeigt eine schematische Darstellung der in Fig. 1 und Fig. 2 beschriebenen Vorrichtung zur Desinfektion einer Person 10. Gut zu erkennen ist hier das erhöhte Geländer 6, welches über einen Lüfter (nicht zu sehen) mit Raumluft versorgt wird. Der Schaltschrank 11 ist zu erkennen und ein schematisch dargestellter Tank 17 zur Aufnahme der Desinfektionsflüssigkeit. In den Tank 17 ist eine Pumpe (nicht dargestellt) zum Versprühen des oxidativ wirkenden Desinfektionsmittels integriert.

kann die Vorrichtung 20 Bodenunebenheiten ausgleichen und so ebenfalls mobil eingesetzt werden.

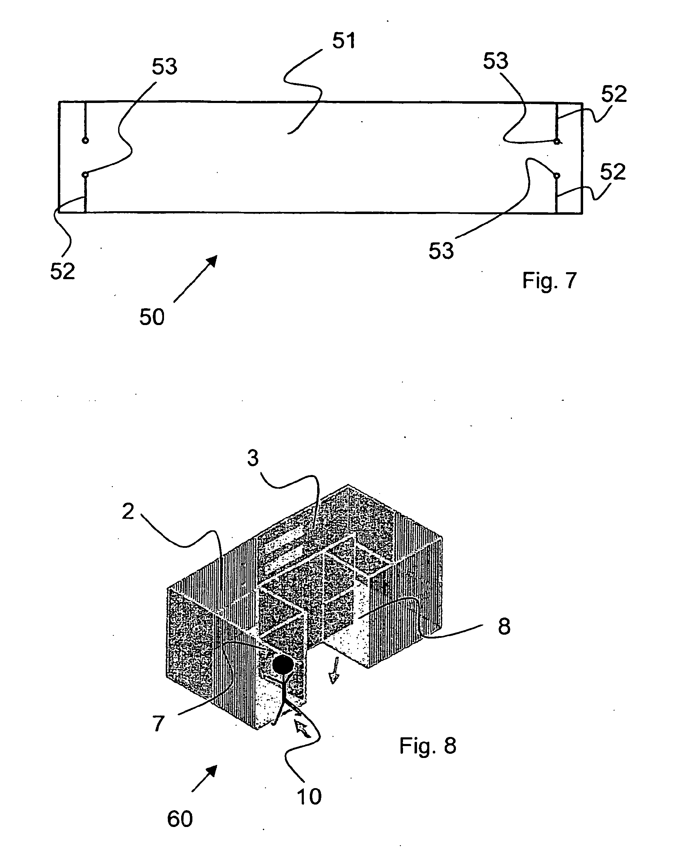

Fig. 7 zeigt eine schematische Darstellung einer Brille 50, die dazu bestimmt ist, bei dem Verfahren unter Verwendung der erfindungsgemäßen Vorrichtung getragen zu werden. Die Brille 50 besteht aus einer rechteckigen Folie 51. Die Folie ist aus einem UV-absorbierenden Kunststoff ausgestaltet. Sie weist randseitig Schlitze 52 mit Aussparungen 53 auf. Durch die Schlitze kann ein Gummiband (nicht dargestellt) geführt werden, das dann in den Aussparungen 53 fixiert ist. So kann die Brille 50 getragen werden. Aufgrund der Flexibilität schließt sie durch den Zug des Gummibandes bündig im Stirnbereich ab, so dass der Träger von oben keine Tropfen in die Augen bekommen kann.

Fig. 8 zeigt eine weitere Ausführungsform einer erfindungsgemäßen Desinfektionseinrichtung für Personen. Die Vorrichtung 60 weist einen Eingangsbereich 7 auf, durch den die Person 10 in den ersten Bereich 2 der Vorrichtung tritt, dort mit einer oxidativ wirkenden Flüssigkeit besprüht wird, sodann im zweiten Bereich 3 mit UV-Licht bestrahlt wird und über einen abgewinkelten Ausgangsbereich 8 die Vorrichtung wieder verlässt. Durch die Ausgestaltung des Eingangsbereiches 7 und des Ausgangsbereiches 8 ist gesichert, dass Personen von außen nicht in das UV-Licht blicken können.

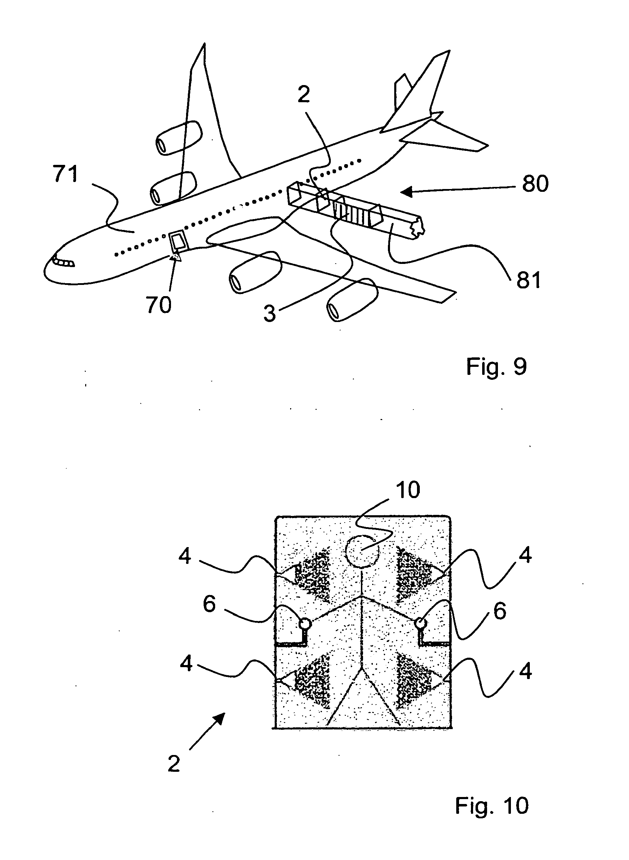

Fig. 9 zeigt schematische Darstellungen von Ausführungsformen der erfindungsgemäßen Vorrichtung.
Zum einen ist hier eine Vorrichtung 70 schematisch dargestellt, die in den Eingangsbereich eines Flugzeuges 71 integriert ist. Zum anderen schematisch dargestellt ist eine Vorrichtung 80, die in eine sogenannte Brücke 81, die auf Flughäfen zum Verlassen eines Flugzeuges 71 vorgesehen ist, integriert ist. Die Brücke 81 ist hier am Flugzeug 71 angedockt. Verlassen nun die Passagiere das Flugzeug 71, durchlaufen sie zunächst den ersten Bereich 2 der Vorrichtung 80, indem sie mit einem oxidativ wirkenden Mittel besprüht werden und durchlaufen sodann den zweiten Bereich 3, in dem sie mit UV-Strahlung bestrahlt werden. Mit dieser Vorrichtung ist es möglich, auf schnelle und effektive Weise alle Passagiere eines Flugzeuges beim Verlassen im Vorbeigehen zu desinfizieren.

Fig. 10 zeigt eine schematische Darstellung des ersten Bereiches 2 einer erfindungsgemäßen Vorrichtung. Zu erkennen sind beidseitig je zwei Sprühköpfe 4, über die die Person 10 mit einem Nebel aus einer oxidativ wirkenden Flüssigkeit besprüht wird. Zu erkennen ist ferner ein beidseitig angeordnetes Geländer 6, das so hoch angeordnet ist, dass die zu desinfizierende Person 10 die Arme anwinkeln muss.

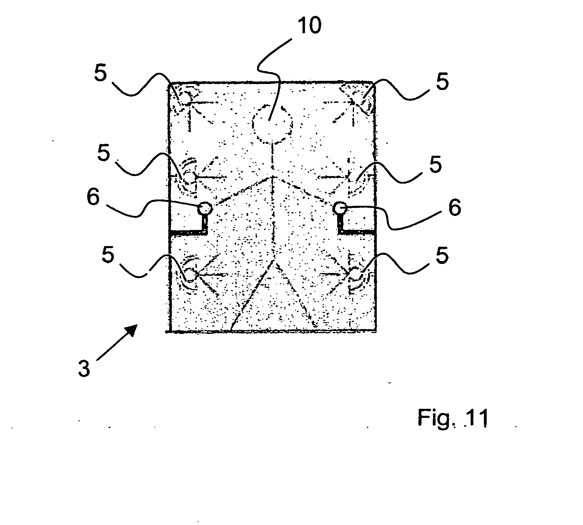

Fig. 11 zeigt eine schematische Darstellung des zweiten Bereiches 3 einer erfindungsgemäßen Vorrichtung. Zu erkennen sind beidseitig je drei UV-Lichtquellen 5, über die die Person 10 mit UV-Licht bestahlt wird wird. Zu erkennen ist auch hier das beidseitig angeordnete Geländer 6.

## Patentansprüche

1. Vorrichtung (1) zur Desinfektion, Abtötung oder Passivierung pathogener Erreger an Menschen umfassend
- einen ersten Bereich (2) mit zumindest einer Vorrichtung zum Versprühen einer Flüssigkeit, die ein oxidativ wirkendes Mittel enthält,
- einen zweiten Bereich (3) mit zumindest einer UV-Lichtquelle (5),
wobei die Vorrichtung auf einer Seite einen Eingang (7) und auf der gegenüber liegenden Seite einen Ausgang (8) sowie
und zumindest im ersten Bereich ein beidseitiges Geländer (6) aufweiset, welches derart ausgebildet ist, dass ein zu desinfizierender Mensch die Arme abwinkeln muss.

2. Vorrichtung nach dem vorhergehenden Anspruch, **dadurch gekennzeichnet, dass** der erste Bereich (2) vom dem zweiten Bereich (3) räumlich nicht getrennt ist.

3. Vorrichtung nach einem der vorhergehenden Ansprüche, **dadurch gekennzeichnet, dass** die Vorrichtung zum mobilen Transport ausgebildet ist.

4. Vorrichtung nach einem der vorhergehenden Ansprüche, **dadurch gekennzeichnet, dass** die Vorrichtung demontierbar ist.

5. Vorrichtung nach einem der vorhergehenden Ansprüche, **dadurch gekennzeichnet, dass** die Vorrichtung teleskopartig zusammenschiebbar ist, insbesondere, dass der zweite Bereich (3) zumindest teilweise in den ersten (2) Bereich einschiebbar ist.

6. Vorrichtung nach einem der vorhergehenden Ansprüche, **dadurch gekennzeichnet, dass** der erste Bereich (2) im wesentlichen aus Edelstahl aufgebaut ist.

7. Vorrichtung nach einem der vorhergehenden Ansprüche, **dadurch gekennzeichnet, dass** der zweite Bereich (3) im wesentlichen aus Edelstahl und/oder Aluminium aufgebaut ist.

8. Vorrichtung nach einem der vorhergehenden Ansprüche, **dadurch gekennzeichnet, dass** die Vorrichtung durch Türen an jeweils dem ersten und/oder zweiten Bereich verschließbar ist.

9. Vorrichtung nach dem vorhergehenden Anspruch, **dadurch gekennzeichnet, dass** die Türen über zumindest einen Sensor automatisch steuerbar sind.

10. Vorrichtung nach einem der vorhergehenden Ansprüche, **dadurch gekennzeichnet, dass** die Vorrichtung einen Sensor zur Steuerung des Sprühvorgangs und/oder einen Sensor zur Steuerung der UV-Bestrahlung aufweist.

11. Vorrichtung nach dem vorstehenden Anspruch, **dadurch gekennzeichnet, dass** der Sensor vor direkter UV-Bestrahlung geschützt angeordnet ist.

12. Vorrichtung nach einem der vorhergehenden Ansprüche, **dadurch gekennzeichnet, dass** zumindest ein Sensor vorgesehen ist, der das Erreichen des Menschen oder Tieres des ersten Bereiches (2), des zweiten Bereiches (3) und/oder das Verlassen der Vorrichtung erkennt.

13. Vorrichtung nach einem der vorhergehenden Ansprüche, **dadurch gekennzeichnet, dass** die Vorrichtung mit einer akustischen und/oder optischen Führungseinrichtung (14) ausgestattet ist.

14. Vorrichtung nach einem der vorhergehenden Ansprüche, **dadurch gekennzeichnet, dass** die Vorrichtung eine Einrichtung zum automatischen Abschalten des Sprühvorgangs und/oder der UV-Bestahlung nach einer vorgegebenen Zeit aufweist.

15. Vorrichtung nach einem der vorhergehenden Ansprüche, **dadurch gekennzeichnet, dass** die Vorrichtung einen Transponder für eine Karte, insbesondere eine Strichcode, Magnet- oder Chipkarte aufweist.

16. Vorrichtung nach einem der vorhergehenden Ansprüche, **dadurch gekennzeichnet, dass** die Vorrichtung ein Förderband (21) für die Menschen aufweist.

17. Vorrichtung nach einem der vorhergehenden Ansprüche, **dadurch gekennzeichnet, dass** erster (2) und zweiter (3) Bereich nacheinander angeordnet sind und keine Sichtschutzwand zwischen erstem und zweiten Bereich vorgesehen ist.

18. Vorrichtung nach dem vorstehenden Anspruch, **dadurch gekennzeichnet, dass** gegenüber dem Eingang (7) und/oder Ausgang (8) eine Sichtschutzwand oder ein Sichtschutzvorhang (9) angeordnet ist.

19. Vorrichtung nach Anspruch 32 oder 33, **dadurch gekennzeichnet, dass** die Sichtschutzwand oder der Sichtschutzvorhang (9) zurückfahrbar oder zurückschiebbar ausgebildet ist.

20. Vorrichtung nach einem der vorhergehenden Ansprüche, **dadurch gekennzeichnet, dass** die Vorrichtung ein Geländer aufweist, durch das über einen Lüfter Raumluft leitbar ist.

21. Vorrichtung nach einem der vorhergehenden Ansprüche, **dadurch gekennzeichnet, dass** der zweite Bereich (3) eine wesentlich größere Länge als der erste Bereich (2) hat.

22. Vorrichtung nach einem der vorhergehenden Ansprüche, **dadurch gekennzeichnet, dass** die Vorrichtung eine unabhängige Stromversorgung zum Betrieb der UV-Lichtquelle (5) aufweist.

23. Vorrichtung nach einem der vorhergehenden Ansprüche, **dadurch gekennzeichnet, dass** die Vorrichtung als Schleuse für einen Operationssaal ausgebildet ist.

24. Vorrichtung nach einem der vorhergehenden Ansprüche, **dadurch gekennzeichnet, dass** die Vorrichtung derart ausgebildet ist, um sie in einer Brücke, die auf einem Flughafen an ein Flugzeug andockt, vorzusehen.

25. Vorrichtung nach einem der vorhergehenden Ansprüche, **dadurch gekennzeichnet, dass** die Vorrichtung als aufblasbares Tor, welches an einem Fortbewegungsmittel, insbesondere einem Flugzeug (71) oder einem Fahrzeug zum Transport von kranken Menschen oder Tieren anbringbar ist, ausgebildet ist.

## Claims

1. Device (1) for disinfecting, eliminating or passivating pathogenic agents on people, including
- a first region (2) having at least one device for spraying a liquid which contains an oxidatively active medium,
- a second region (3) having at least one UV light source (5),
wherein the device comprises an entrance (7) on one side and an exit (8) on the opposite side as well as a handrail (6) on both sides at least in the first region, which handrail is formed such that a person to be disinfected has to bend his arm.

2. Device as claimed in the preceding Claim, **characterised in that** the first region (2) is not spatially separated from the second region (3).

3. Device as claimed in any one of the preceding Claims, **characterised in that** the device is formed for mobile transport.

4. Device as claimed in any one of the preceding Claims, **characterised in that** the device can be disassembled.

5. Device as claimed in any one of the preceding Claims, **characterised in that** the device is telescopic, in particular **in that** the second region (3) can be pushed at least partially into the first region (2).

6. Device as claimed in any one of the preceding Claims, **characterised in that** the first region (2) is constructed substantially from stainless steel.

7. Device as claimed in any one of the preceding Claims, **characterised in that** the second region (3) is constructed substantially from stainless steel and/or aluminium

8. Device as claimed in any one of the preceding Claims, **characterised in that** the device can be closed by doors in each case at the first and/or second region.

9. Device as claimed in the preceding Claim, **characterised in that** the doors can be controlled automatically by at least one sensor.

10. Device as claimed in any one of the preceding Claims**, characterised in that** the device comprises a sensor for controlling the spraying process and/or a sensor for controlling the UV irradiation.

11. Device as claimed in the preceding Claim, **characterised in that** the sensor is disposed so as to be protected from direct UV irradiation.

12. Device as claimed in any one of the preceding Claims, **characterised in that** at least one sensor is provided which recognises when the person or animal arrives at the first region (2), the second region (3) and/or when he/it exits the device.

13. Device as claimed in any one of the preceding Claims, **characterised in that** the device is fitted with an acoustic and/or optical guide unit (14).

14. Device as claimed in any one of the preceding Claims, **characterised in that** the device comprises a unit for automatically discontinuing the spraying process and/or the UV irradiation after a predetermined length of time.

15. Device as claimed in any one of the preceding Claims, **characterised in that** the device comprises a transponder for a card, in particular a bar code, magnetic card or chip card.

16. Device as claimed in any one of the preceding Claims, **characterised in that** the device comprises a conveyor belt (21) for the people.

17. Device as claimed in any one of the preceding Claims, **characterised in that** the first (2) and second (3) regions are successively disposed and there is no screen wall between the first and second regions.

18. Device as claimed in the preceding Claim, **characterised in that** a screen wall or a screen curtain (9) is disposed opposite the entrance (7) and/or the exit (8).

19. Device as claimed in Claim 32 or 33 [*sic*], **characterised in that** the screen wall or the screen curtain (9) is formed so as to be able to be retracted or pushed back.

20. Device as claimed in any one of the preceding Claims, **characterised in that** the device comprises a handrail, through which ambient air can be conducted via an aerator.

21. Device as claimed in any one of the preceding Claims, **characterised in that** the second region (3) is substantially longer in length than the first region (2).

22. Device as claimed in any one of the preceding Claims, **characterised in that** the device comprises an independent power supply for operating the UV light source (5).

23. Device as claimed in any one of the preceding Claims, **characterised in that** the device is formed as a decontamination area for an operating theatre.

24. Device as claimed in any one of the preceding Claims, **characterised in that** the device is formed in such a manner for it to be provided in a bridge which is connected to an aeroplane in an airport.

25. Device as claimed in any one of the preceding Claims, **characterised in that** the device is formed as an inflatable gateway which can be attached to a means for locomotion, in particular an aeroplane (71) or a vehicle for transporting ill people or animals.

## Revendications

1. Dispositif (1) destiné à la désinfection, à la destruction ou à la passivation d'agents pathogènes sur les êtres humains, comprenant :
- une première zone (2) avec au moins un dispositif de pulvérisation d'un liquide contenant un agent à action oxydante,
- une deuxième zone (3) avec au moins une source de lumière ultraviolette (5),
dans lequel le dispositif présente d'un côté une entrée (7) et du côté opposé une sortie (8) ainsi que
au moins une balustrade (6) de part et d'autre dans la première zone, qui est configurée de telle manière que la personne à désinfecter doit ouvrir l'angle des bras.

2. Dispositif selon la revendication précédente, **caractérisé en ce que** la première zone (2) n'est pas séparée spatialement de la deuxième zone (3).

3. Dispositif selon l'une des revendications précédentes, **caractérisé en ce que** le dispositif est conçu pour être transporté.

4. Dispositif selon l'une des revendications précédentes**, caractérisé en ce que** le dispositif est démontable.

5. Dispositif selon l'une des revendications précédentes, **caractérisé en ce que** le dispositif peut se rétracter de manière télescopique, en particulier **en ce que** la deuxième zone (3) peut se rétracter au moins partiellement dans la première zone (2).

6. Dispositif selon l'une des revendications précédentes, **caractérisé en ce que** la première zone (2) est pour l'essentiel construite en acier inoxydable.

7. Dispositif selon l'une des revendications précédentes, **caractérisé en ce que** la deuxième zone (3) est pour l'essentiel construite en acier inoxydable et/ou en aluminium.

8. Dispositif selon l'une des revendications précédentes, **caractérisé en ce que** le dispositif peut être fermé par des portes placées sur la première et/ou la deuxième zone.

9. Dispositif selon la revendication précédente, **caractérisé en ce que** les portes peuvent être commandées automatiquement à l'aide d'au moins un capteur.

10. Dispositif selon l'une des revendications précédentes, **caractérisé en ce que** le dispositif possède un capteur destiné à commander l'opération de pulvérisation et/ou un capteur destiné à commander l'irradiation ultraviolette.

11. Dispositif selon la revendication précédente, **caractérisé en ce que** le capteur est disposé de manière à être protégé du rayonnement ultraviolet direct.

12. Dispositif selon l'une des revendications précédentes, **caractérisé en ce qu'**il est prévu au moins un capteur qui détecte l'arrivée d'un être humain ou d'un animal dans la première zone (2), dans la deuxième zone (3) et/ou à la sortie du dispositif.

13. Dispositif selon l'une des revendications précédentes, **caractérisé en ce que** le dispositif est équipé d'un équipement de guidage (14) acoustique et/ou optique.

14. Dispositif selon l'une des revendications précédentes, **caractérisé en ce que** le dispositif possède un équipement de coupure automatique de l'opération de pulvérisation et/ou de l'irradiation ultraviolette après une période de temps prédéterminée.

15. Dispositif selon l'une des revendications précédentes, **caractérisé en ce que** le dispositif possède un transpondeur pour une carte, en particulier un code à barres, une carte magnétique ou une carte à puce.

16. Dispositif selon l'une des revendications précédentes, **caractérisé en ce que** le dispositif possède une bande transporteuse (21) pour êtres humains.

17. Dispositif selon l'une des revendications précédentes, **caractérisé en ce que** la première (2) et la deuxième (3) zone se succèdent l'une à l'autre et **en ce qu'**aucune cloison de protection oculaire n'est prévue entre la première et la deuxième zone.

18. Dispositif selon la revendication précédente, **caractérisé en ce que**, en face de l'entrée (7) et/ou de la sortie (8), il est prévu une cloison de protection oculaire ou un rideau de protection oculaire (9).

19. Dispositif selon la revendication 32 ou 33,
**caractérisé en ce que** la paroi de protection oculaire ou le rideau de protection oculaire (9) est construit de manière à pouvoir être escamoté ou repoussé.

20. Dispositif selon l'une des revendications précédentes, **caractérisé en ce que** le dispositif possède une balustrade grâce à laquelle peut être guidé de l'air ambiant envoyé par un ventilateur.

21. Dispositif selon l'une des revendications précédentes, **caractérisé en ce que** la deuxième zone (3) possède une longueur nettement plus grande que la première zone (2).

22. Dispositif selon l'une des revendications précédentes, **caractérisé en ce que** le dispositif possède une alimentation électrique indépendante afin d'alimenter la source de lumière ultraviolette (5).

23. Dispositif selon l'une des revendications précédentes, **caractérisé en ce que** le dispositif est conçu comme un sas d'une salle d'opération.

24. Dispositif selon l'une des revendications précédentes, **caractérisé en ce que** le dispositif est configuré de manière à l'installer dans une passerelle qui, sur un aéroport, vient au contact d'un aéronef.

25. Dispositif selon l'une des revendications précédentes, **caractérisé en ce que** le dispositif est configuré sous la forme d'un portail gonflable, de manière à pouvoir être appliqué à un moyen de transport, en particulier un aéronef (71) ou un véhicule de transport d'êtres humains ou d'animaux malades.
